# EUROPEAN PATENT APPLICATION

(11) **EP 1 043 300 A2**
(43) Date of publication of application: **11.10.2000**
(21) Application number: 00302351.2
(22) Date of filing: 22.03.2000
(51) Int. Cl.: C07C 51/12, C07C 53/08, C07C 67/36, C07C 69/14

(54) **Process for the carbonylation of an alcohol and/or reactive derivative thereof**

(30) Priority: 06.04.1999 GB 9907834
(71) Applicant: BP Chemicals Limited, London EC2M 7BA (GB)
(72) Inventor: Ditzel, Evert Jan, Hull, HU12 8DS (GB); Poole, Andrew David, Hull, HU12 8DS (GB); Cole-Hamilton, David John, Fife, KY16 9ST (GB); Marr, Andrew Craig, Nottingham, N47 1LP (GB)
(74) Representative: Perkins, Nicholas David

(57) **Abstract**

A process for the production of a carboxylic acid which process comprises contacting a liquid reaction composition comprising an alcohol and/or a reactive derivative thereof, a hydrocarbyl halide co-catalyst and optionally water with carbon monoxide, optionally with hydrogen at a molar ratio of hydrogen : carbon monoxide of less than 1 : 10, in the presence of a cobalt catalyst precursor having the following structural formula (I) : 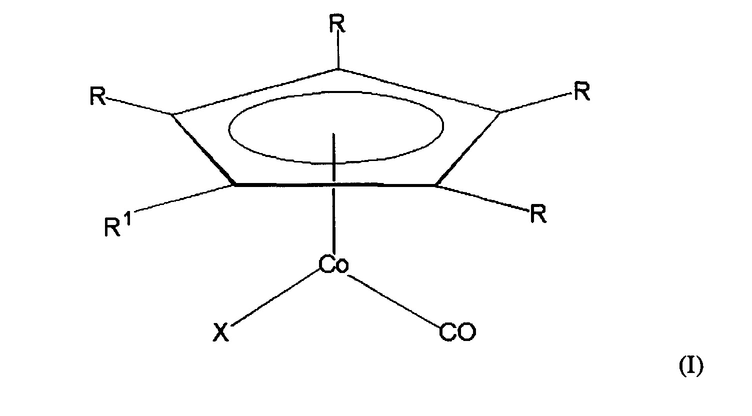 wherein :
the R groups are independently hydrogen, alkyl, aryl, substituted alkyl or aryl; the R groups may be the same or different to each other;
R¹ is a hydrogen, alkyl, aryl, substituted alkyl or aryl or a bridging group to X; and
X is a two electron donor which may optionally be linked to the cyclopentadienyl group via R¹
and the Co may additionally be bound to a Z group such that Z is halide, in which case in place of the CO group there may be an acyl group of formula R⁴CO in which R⁴ is hydrogen, alkyl, aryl or substituted alkyl or aryl.

## Description

The present invention relates to a process for the carbonylation of an alcohol and/or a reactive derivative thereof and in particular to a process carried out in the presence of a cobalt catalyst.

Acetic acid is a known and valuable industrial chemical. Acetic acid is generally prepared by carbonylating methanol or a reactive derivative in the presence of a Group VIII metal catalyst. US 3769329 and GB 1233121 for example, disclose a process for the carbonylation of methanol using rhodium catalysts.

It is also known to produce acetic acid by carbonylation of methanol in the presence of a cobalt catalyst. Such a process is described in Howard et al in *Catalysis Today* 18 (1993) 325-354 in section 2.1 at page 326. Traditionally, the commercial cobalt catalysed carbonylation process requires high pressure and high temperature. We have now found that acetic acid can be produced through the use of a cobalt cyclopentadienyl catalyst which allows less severe conditions.

EP-A- 0029723 describes the use of cyclopentadienyl complexes of Group VIII metal other than iron, palladium and platinum in homologation reactions of methanol with synthesis gas ( a mixture of hydrogen and carbon monoxide) to produce ethanol and/or acetaldehyde. According to EP-A-0029723 the molar ratio of carbon monoxide : hydrogen is suitably in the range 2:1 to 1:3. The present invention is a carbonylation process which does not use significant amounts of hydrogen, if any.

Accordingly, the present invention provides a process for the carbonylation of an alcohol and/or reactive derivative thereof which process comprises contacting a liquid reaction composition comprising an alcohol and/or a reactive derivative thereof, a hydrocarbyl halide co-catalyst and optionally water, with carbon monoxide, optionally with hydrogen at a molar ratio of hydrogen : carbon monoxide of less than 1 : 10, in the presence of a cobalt catalyst precursor having the following structural formula (I) : 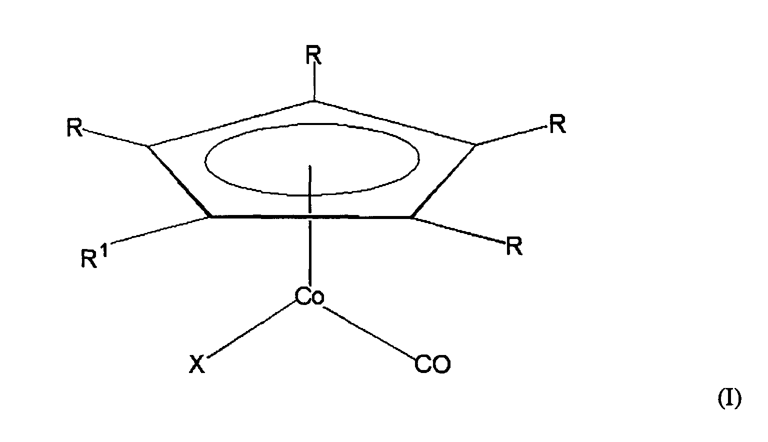 wherein :
the R groups are independently hydrogen, alkyl, aryl, substituted alkyl or aryl; the R groups may be the same or different to each other;
R¹ is a hydrogen, alkyl, aryl, substituted alkyl or aryl or a bridging group to X; and
X is a two electron donor which may optionally be linked to the cyclopentadienyl group via R¹.

In the carbonylation process of the present invention, the alcohol may suitably be an aliphatic alcohol having up to 6 carbon atoms, for example methanol, ethanol, propanols, butanols, pentanols and hexanols. The carbonylation product of an alcohol having n carbon atoms will comprise a carboxylic acid having n + 1 carbon atoms and/or its ester with the alcohol reactant. A preferred alcohol is methanol, the carbonylation product of which comprises acetic acid and/or methyl acetate.

Alternatively or additionally, reactive derivatives of the alcohol may be used, for example alkyl halides, dialkyl ethers and esters of alcohols having n carbon atoms with the carboxylic acid product having n+1 carbon atoms. Suitable reactive derivatives of methanol include, for example, methyl acetate, dimethyl ether and methyl iodide. A mixture of more than one alcohol and/or reactive derivative may be used, for example methanol and methyl acetate.

In the carbonylation process of the present invention, the carboxylic acid ester reactant may suitably be an ester of a C₁ to C₆ carboxylic acid and a C₁ to C₆ monofunctional aliphatic alcohol. Preferably, the ester reactant is an ester of a carboxylic acid and methanol, ethanol or propanol. Particularly preferred, is methyl acetate. A mixture of esters may be employed.

The carbonylation process is carried out in the presence of a hydrocarbyl halide co-catalyst. The hydrocarbyl halide co-catalyst may be a C₁ to C₆ halide for example methyl, ethyl or propyl halide. The halide may be any halide, preferably iodide. The preferred hydrocarbyl halide co-catalyst is methyl iodide. The concentration of hydrocarbyl halide co-catalyst in the reaction composition is suitably in the range of from 1 to 30% by weight, preferably 1 to 20% by weight, most preferably 5 to 20% by weight.

Water may be present in the reaction system, suitably in an amount of greater than 0.1% up to 25% by weight based on the weight of reaction composition. Water is generally produced during the carbonylation process as a by-product of esterification. The water produced, may be recycled to the reaction composition.

The carbonylation process may also be carried out in the presence of an ionic halide promoter, preferably iodide. Suitable promoters may be selected from Group IA and Group IIA metal iodides, quaternary ammonium iodides and phosphonium iodides, optionally prepared in-situ. The concentration of ionic halide promoter in the reaction composition is suitably equivalent to up to 20% by weight halide.

Carbon monoxide used in the process of the present invention, may be essentially pure or may contain inert impurities such as carbon dioxide, methane, nitrogen, noble gases and C₁ to C₄ paraffinic hydrocarbons. Optionally, minor amounts of hydrogen may be present in a molar ratio of hydrogen : carbon monoxide of less than 1 : 10. The presence of hydrogen in the carbon monoxide feed and generated in situ by the water gas shift reaction is preferably kept low, for example, less than 2 bar partial pressure, as its presence may result in the formation of hydrogenation products.

The catalyst used in the process of the present invention is a cobalt-cyclopentadienyl compound. Preferably, the concentration of the cobalt-containing compound in the liquid reaction composition is in the range of from 100 to 20000 ppm by weight of cobalt.

The catalyst precursor used in the process of the present invention is a cyclopentadienyl cobalt complex of formula (I). The R groups of formula (I) are independently selected from hydrogen, alkyl, aryl or substituted alkyl or aryl. Each R may be the same or may be different. Preferably, R is alkyl, more preferably methyl.

The cyclopentadienyl group may be part of a larger ring system, for example indenyl or flourenyl.

R¹ of formula (I) may be hydrogen, alkyl, aryl or substituted alkyl or aryl, for example, methyl. R¹ may be a bridging group to X. Suitably, where R¹ is a bridging group, R¹ may be (CR'''₂)ₙYₘ, wherein the R''' groups are independently hydrogen, alkyl, aryl or substituted alkyl or aryl, and may be the same or different, n is an integer 0 to 5, Y is a group containing silicon, oxygen, nitrogen or sulphur and m is an integer 0 to 5.

X of formula (I) is a two electron donor, for example CO, PR³₃, NR³₃, SR³₂ and OR³₂ where each R³ group is independently hydrogen, alkyl, aryl or substituted alkyl or aryl and the R³ groups which may be the same or different. When R¹ is a bridging group, suitable two electron donors include PR''₂, NR''₂, SR'', OR'' where R'' is alkyl, aryl or substituted alkyl or aryl which may be the same or different. The P, S, N and O atoms may also be part of a ring system, for example an aromatic system such as pyridine. X may also be a charged species, for example a halide, preferably iodide, in which case the catalyst precursor is also charged.

In a further embodiment of the present invention, the Co of formula (I) is additionally bound to a Z group, such that Z = X = halide, preferably iodide. The cobalt catalyst precursor thus has the structural formula (II) : 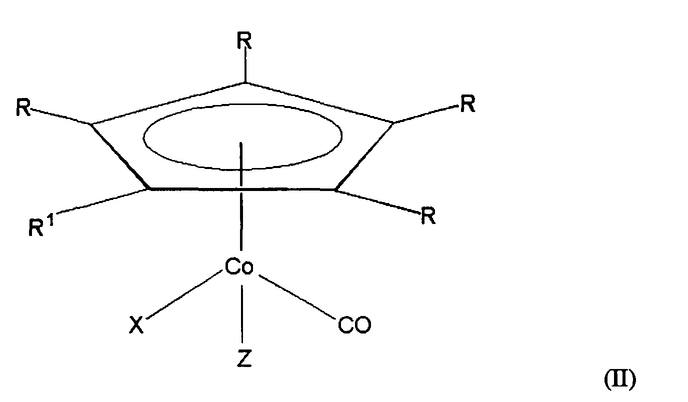 wherein R and R¹ are as hereinbefore defined for formula (I) and X and Z are halide, preferably iodide.

In yet a further embodiment of the present invention, the Co of formula (I) is additionally bound to a Z group, such that Z is halide, preferably iodide and in place of the CO group of formula (I) there is an acyl group of formula R⁴CO in which R⁴ is hydrogen, alkyl, aryl or substituted alkyl or aryl. Preferably R⁴ is alkyl, preferably methyl. The cobalt catalyst precursor thus has the structural formula (III) : 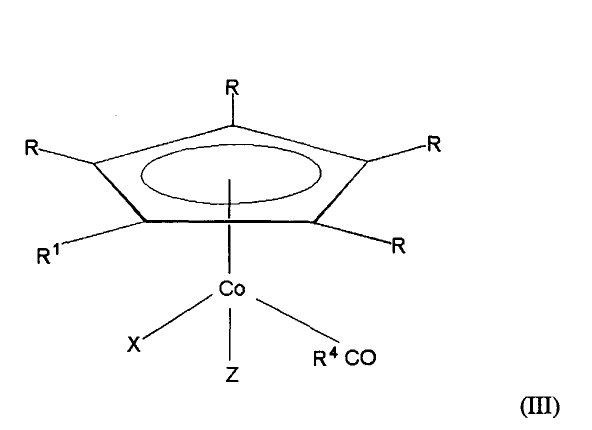 wherein R, R¹ and X are as hereinbefore defined for formulae (I) and (II), Z is halide, preferably iodide and R⁴ is hydrogen, alkyl, aryl or substituted alkyl or aryl. Preferably R⁴ is alkyl, preferably methyl.

The catalyst precursor may be prepared prior to addition to the liquid reaction composition or may be prepared *in situ* in the liquid reaction composition. A suitable method of preparing the catalyst precursor *in situ* is to react in the liquid reaction composition, a cobalt cyclopentadienyl compound of formula (I), (II) or (III) in which X is CO with a reagent capable of substituting the CO with a group having the formula X other than CO. Suitable reagents include PR³₃, NR³₃, SR³₂ and OR³₂.

The carbonylation process may be carried out under a pressure in the range of from 1 to 250 barg, preferably 20 to 200 barg. The carbonylation process may be carried out at a temperature suitably in the range of 60 to 250°C.

The carbonylation process may be carried out as a batch or a continuous process. Preferably, the process is a continuous process.

The carbonylation product comprising carboxylic acid and/or ester thereof may be removed from the reactor by withdrawing the liquid reaction composition and separating the product from the other components in the liquid reaction composition by one or more flash and/or distillation stages. The product may be removed as a vapour from the reactor.

The invention will now be illustrated by way of the following examples in which Me = CH₃ and Et = C₂H₅.

### Example 1 (R and R¹ = Me, X = PEt₃)

A 300 ml Hastelloy B2 autoclave equipped with a dispersimax stirrer, a ballast vessel and a liquid catalyst injection system was used for a series of batch carbonylation experiments. A gas supply to the autoclave was provided from a gas ballast vessel, feed gas being provided to maintain the autoclave at a constant pressure and the rate of gas uptake being calculated (with an accuracy believed to be ± 1%) from the rate at which the pressure falls in the ballast vessel.

The cobalt complex and the liquid charge were weighed out into the autoclave under nitrogen inside a glove bag, and the autoclave sealed. The autoclave was cooled using a dry ice/acetone slush bath, and the PEt₃ injected via one of the ports. The autoclave was then removed from the glove bag and the unit assembled. The autoclave headspace was flushed with carbon monoxide once and then pressurised to about 60 barg with carbon monoxide at room temperature. The autoclave was heated with stirring (1500 ppm) to the desired temperature. Once at temperature the pressure in the autoclave was increased to 100 barg with carbon monoxide. The autoclave pressure was kept constant (± 0.5 barg) using carbon monoxide fed from the ballast vessel.

On completion the autoclave was isolated from the gas supply and the reactor contents cooled to room temperature. The autoclave was vented and the vent gases sampled and analysed. The liquid reaction composition was discharged from the autoclave, sampled and was analysed for liquid products and by-products using gas chromatography. The acetic acid production was calculated on the basis of carbon monoxide uptake and consumption.

### Example 2 (R and R¹ = H and X is PEt₃)

The procedure of Example 1 was repeated except the cobalt complex comprised R and R¹ as hydrogen and X as PEt₃. HPIR confirmed the presence of this complex at the start of the reaction.

### Example 3 and 4 (R and R¹ = Me, X is CO)

The procedure of Example 1 was repeated except the cobalt complex comprised R and R¹ as CH₃ and X as CO.

### Example 5 and 6 (R and R¹ = Me, X = PEt₃)

A 30 ml Hastelloy B2 autoclave equipped with a mechanical stirrer, a ballast vessel and a liquid catalyst injection system was used. A gas supply to the autoclave was provided from a gas ballast vessel, feed gas being provided to maintain the autoclave at a constant pressure and the rate of gas uptake being calculated from the rate at which the pressure falls in the ballast vessel.

The metal complex was weighed into the autoclave under argon. The apparatus was flushed with argon and the liquid charge except methyl iodide injected into the autoclave. The autoclave was pressurised to 60 barg with carbon monoxide and stirred. The autoclave was heated with stirring to 185°C. The methyl iodide was then injected into the autoclave using an overpressure of carbon monoxide. The overpressure was calculated so that on injection the desired reaction pressure was achieved. After catalyst injection the autoclave pressure was kept constant using carbon monoxide fed from the ballast vessel.

On completion the autoclave was isolated from the gas supply and the reactor contents cooled to room temperature. The autoclave was vented degassed and the liquid reaction composition was discharged from the autoclave.

The autoclave charges relating to each example are given in Table 1 in which Cp is cyclopentadienyl ring and Cp* is pentamethyl cyclopentadienyl ring, MeOH represents methanol, MeI represent methyl iodide, MeOAc represents methyl acetate and AcOH represents acetic acid. Water was not added in Examples 1 to 3 but would be formed in situ. The reaction rates and turnovers are also given in Table 1.

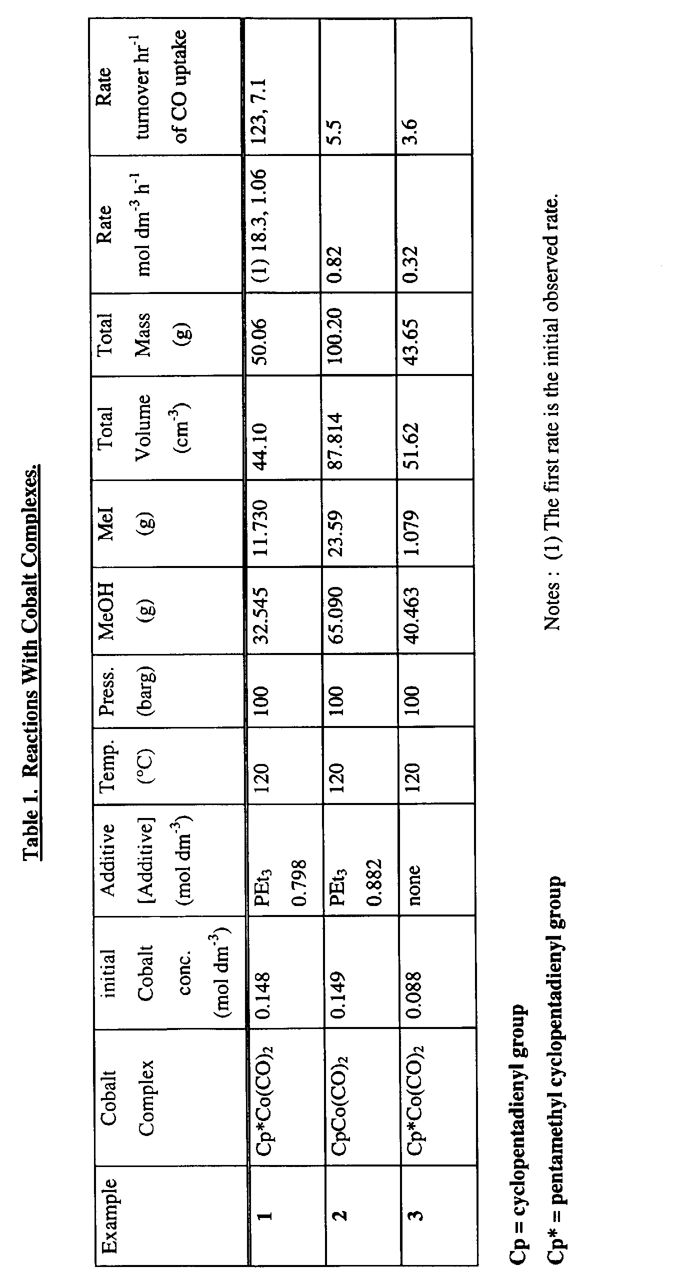

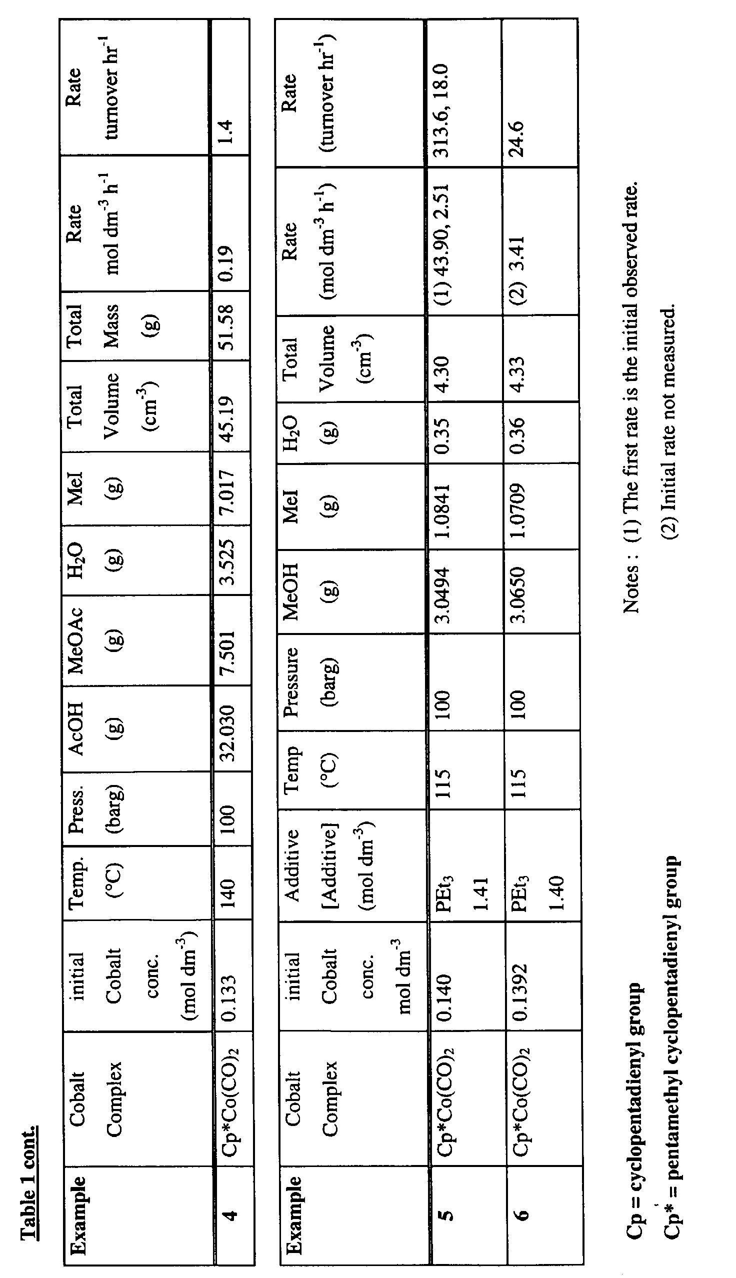

## Claims

1. A process for the carbonylation of an alcohol and/or reactive derivative thereof which process comprises contacting a liquid reaction composition comprising an alcohol and/or a reactive derivative thereof, a hydrocarbyl halide co-catalyst and optionally water with carbon monoxide, optionally with hydrogen at a molar ratio of hydrogen : carbon monoxide of less than 1 : 10, in the presence of a cobalt catalyst precursor having the following structural formula (I) : 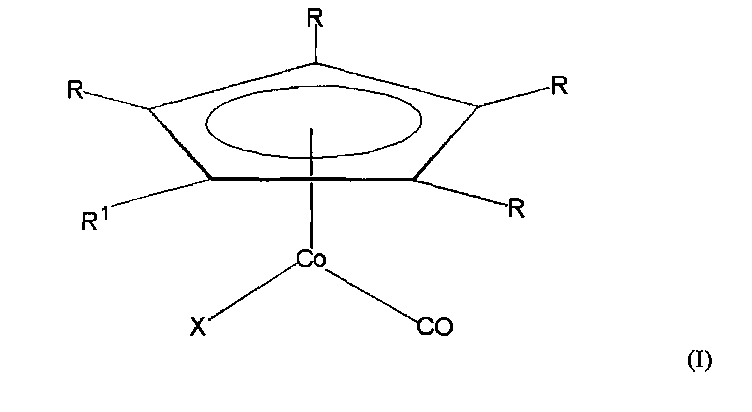 wherein :
the R groups are independently hydrogen, alkyl, aryl, substituted alkyl or aryl; the R groups may be the same or different to each other;
R¹ is a hydrogen, alkyl, aryl, substituted alkyl or aryl or a bridging group to X; and
X is a two electron donor which may optionally be linked to the cyclopentadienyl group via R¹.

2. A process as claimed in claim 1 in which in formula (I) R is alkyl, preferably methyl.

3. A process as claimed in claim 1 in which the cyclopentadienyl group is part of a larger ring system, preferably indenyl or flourenyl.

4. A process as claimed in any one of the preceding claims in which in formula (I) R¹ is methyl.

5. A process as claimed in any one of claims 1 to 3 in which in formula (I) the R¹ group is a bridging group to X.

6. A process as claimed in claim 5 in which R¹ has the formula (CR'''₂)ₙYₘ, wherein the R''' groups are independently hydrogen, alkyl, aryl or substituted alkyl or aryl, and may be the same or different, n is an integer 0 to 5, Y is a group containing silicon, oxygen, nitrogen or sulphur and m is an integer 0 to 5.

7. A process as claimed in claim 5 or claim 6 in which in formula (I) X is PR''₂, NR''₂, SR'', OR'' where R'' is alkyl, aryl or substituted alkyl or aryl which may be the same or different.

8. A process as claimed in any one of claims 1 to 4 in which in formula (I) X is CO, PR³₃, NR³₃, SR³₂ or OR³₂ in which each R³ group is independently hydrogen, alkyl, aryl or substituted alkyl or aryl and the R³ groups which may be the same or different.

9. A process as claimed in any one of claims 7 and 8 in which in X the P, S, N and O atoms are part of a ring system.

10. A process as claimed in claim 9 in which the ring system is an aromatic system, preferably pyridine.

11. A process as claimed in any one of claims 1 to 4 in which X is a charged species, preferably a halide.

12. A process as claimed in any one of claims 1 to 11 modified in that the Co of formula (I) is additionally bound to a Z group, such that Z = X = halide, preferably iodide.

13. A process as claimed in any one of claims 1 to 11 modified in that the Co of formula (I) is additionally bound to a Z group, such that Z is halide, preferably iodide and in place of the CO group of formula (I) there is an acyl group of formula R⁴CO in which R⁴ is hydrogen, alkyl, aryl or substituted alkyl or aryl.

14. A process as claimed in claim 13 in which R⁴ is alkyl, preferably methyl.

15. A process as claimed in claim 1 in which R, R¹ and X in formula (I) are selected from the group consisting of (a) R=R¹=CH₃ and X = P(C₂H₅)₃; (b) R=R¹=H and X = P(C₂H₅)₃ and (c) R=R¹=CH₃ and X = CO.

16. A process as claimed in any one of the preceding claims in which the partial pressure of hydrogen, if present, is less than 2 bar.

17. A process as claimed in any one of the preceding claims in which the alcohol is methanol and there is produced acetic acid and/or methyl acetate.

18. A process as claimed in any one of the preceding claims in which the concentration of hydrocarbyl halide co-catalyst in the reaction composition is in the range 1 to 30 % by weight, the concentration of water in the reaction composition is in the range of greater than 0.1 % up to 25 % by weight, and the concentration of cobalt-containing compound in the reaction composition is in the range of from 100 to 20000 ppm by weight cobalt.
